# EUROPÄISCHE PATENTSCHRIFT

(11) **EP 1 820 731 B1**
(45) Veröffentlichungstag und Bekanntmachung des Hinweises auf die Patenterteilung: **28.10.2009**
(21) Anmeldenummer: 07003481.4
(22) Anmeldetag: 20.02.2007
(51) Int. Cl.: B65B 55/10

(54) **Verfahren zum Sterilisieren von Flaschen oder dergleichen Behältern sowie Vorrichtung zum Durchführen dieses Verfahrens**
Method for sterilising bottles or similar containers and device for performing this method
Procédé de stérilisation de bouteilles ou récipients analogues tout comme dispositif d'exécution de ce procédé

(30) Priorität: 21.02.2006 DE 102006007944
(43) Veröffentlichungstag der Anmeldung: 22.08.2007
(73) Patentinhaber: KHS AG, 44143 Dortmund (DE)
(72) Erfinder: Till, Volker, 65719 Hofheim/Taunus (DE); Sangi, Daryoush, 20251 Hamburg (DE)

(56) Entgegenhaltungen:
- EP-A1- 0 381 841
- EP-A1- 1 086 896
- WO-A-01/78791
- WO-A1-00/00394
- WO-A1-03/070024
- DE-B- 1 191 676

## Beschreibung

Die Erfindung bezieht sich auf ein Verfahren gemäß Oberbegriff Patentanspruch1 sowie auf eine Vorrichtung zum Durchführen des Verfahrens gemäß Oberbegriff Patentanspruch 7.

Die Sterilisierung von Flaschen oder dergleichen Behälter durch Aufbringen von Wasserstoffperoxid (H₂O₂) auf die Innenflächen des zu sterilisierenden Behälters z.B. unter Verwendung eines Behandlungsmediums in Form eines Aerosols aus steriler Luft und dem Wasserstoffperoxid und durch anschließendes Beaufschlagen der Behälterinnenflächen mit heißer steriler Luft zur Erwärmung des jeweiligen Behälters und zur Aktivierung des Sterilisationsmediums sowie zum Entfernen dieses Mediums und eventueller Restwassermengen am Ende der Sterilisationsphase bzw. Sterilisationsprozesses ist bekannt.

Das Einbringen der Behandlungsmedien erfolgt hierbei über eine Behandlungsdüse, die eine einzige auf den Boden des jeweiligen Behälters gerichtete Düsenöffnung aufweist, sodass das jeweilige Behandlungsmedium u. a. nach dem Auftreffen auf den Behälterboden an der Behälterinnenfläche nach oben strömt und sich dadurch gleichmäßig an den Innenflächen des Behälters verteilt.

Die Wirksamkeit des Sterilisationsverfahrens hängt von einer optimierten Strömung des jeweiligen Behandlungsmediums ab, d. h. die Strömung des Behandlungsmediums muss so erfolgen, dass sämtliche Bereiche der Behälterinnenfläche von dem Behandlungsmedium erfasst, bzw. mit diesem beaufschlagt werden.

Weisen Flaschen oder dergleichen Behälter eine von der üblichen, kontinuierlichen Form abweichende Innenkontur auf, insbesondere in der Weise, dass durch die Innenkontur im Inneren des Behälters Erweiterungen und/oder schwerer zugängliche oder strömungsungünstige Nebenräume gebildet sind, so ist mit den bisherigen Verfahren und den hierbei verwendeten Mitteln eine wirksame Sterilisation nicht möglich.

Aufgabe der Erfindung ist es, ein Verfahren aufzuzeigen, welches es ermöglicht, auch Flaschen oder dergleichen Behälter mit einer von der üblichen Form abweichenden und Erweiterungen und/oder Nebenräume usw. bildenden Innenkontur in optimaler Weise zu sterilisieren. Zur Lösung dieser Aufgabe ist ein Verfahren entsprechend dem Patentanspruch 1 ausgebildet. Eine Vorrichtung ist Gegenstand des Patentanspruchs 7.

Die Erfindung wird im Folgenden anhand der Figuren an Ausführungsbeispielen näher erläutert. Es zeigen:
- Fig. 1: in vereinfachter schematischer Darstellung eine Flasche mit einer von der üblichen Flaschenform abweichenden Formgebung, zusammen mit einer in die Flasche eingeführten Behandlungsdüse zum Einbringen eines Behandlungsmediums in den Innenraum der Flasche während der Flaschensterilisation;
- Fig. 2: einen Teilschnitt durch die Düse der Figur 1;
- Fig. 3: in perspektivischer Darstellung die Behandlungsdüse der Figur 1;
- Fig. 4: in einer Darstellung wie Figur 1 eine weitere Ausführungsform die nicht gemäß Erfindung ist;
- Fig. 5: einen Schnitt durch die Behandlungsdüse der Figur 4.

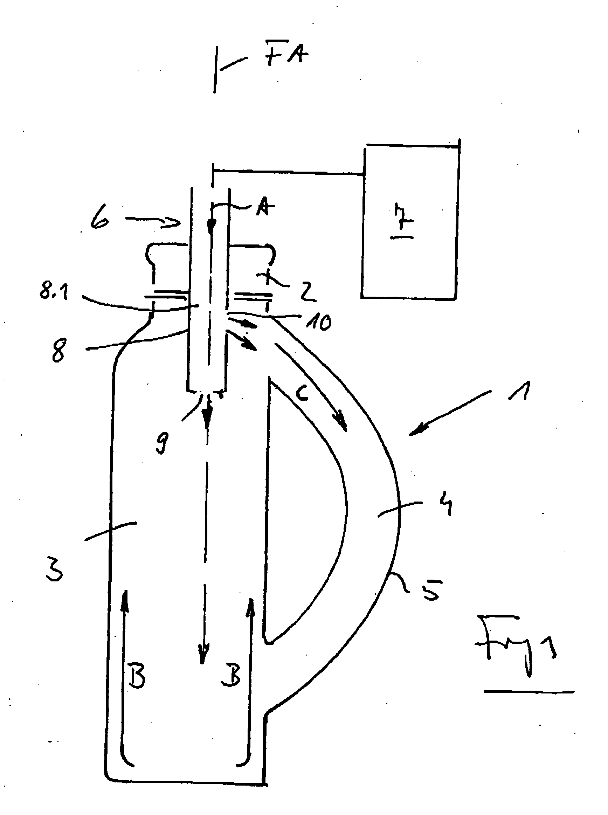

In der Figur 1 ist 1 eine Flasche bzw. ein flaschenartiger Behälter mit einer besonderen Formgebung, und zwar in der Weise, dass der Innenraum bzw- das Volumen der Flasche 2 nicht nur von dem sich axial an die Flaschenmündung 2 anschließenden Flaschenraum 3, sondern auch von einem Teil- oder Nebenraum gebildet ist, nämlich von dem Innenraum 4 eines an die Flasche 1 angeformten hohlen Handgriffes 5.

Um während des Sterilisationsprozesses eine ausreichende Behandlung nicht nur des Flaschenraumes 3, sondern auch des Teil- oder Nebenraumes 4 mit den Behandlungsmedien (zunächst mit Aerosol aus steriler Luft und Wasserstoffperoxid und anschließend mit heißer steriler Luft) zu gewährleisten, wird zum Einbringen der Sterilisations- oder Behandlungsmedien eine Behandlungsdüse 6 verwendet, die Teil einer in der Figur 1 schematisch mit dem Block 7 angedeuteten Behandlungs- oder Sterilisationsvorrichtung ist und die mit ihrem rohrartigen Düsenkörper 8 durch die Flaschenmündung 2 in die Flasche 1 eingeführt ist, und zwar beispielsweise derart, dass die Achse des Düsenkörpers 8 achsgleich mit der Flaschenachse FA liegt.

Die Behandlungs- und Sterilisationsvorrichtung 7 weist dann beispielsweise - abgesehen von der speziellen Ausbildung der Behandlungsdüse 6 - den dem Fachmann bekannten Aufbau einer solchen Vorrichtung auf.

Die Behandlungsmedien werden dem Düsenkörper 8 bzw. dem in diesem Körper ausgebildeten Düsenkanal 8.1 entsprechend dem Pfeil A zugeführt. An dem freien Ende bildet der Düsenkörper 8 zumindest eine Düsenöffnung 9 für den Austritt der Behandlungsmedien, insbesondere zur Behandlung des Flaschenraumes 3 bzw. der diesen Raum begrenzenden Innenflächen der Flasche 1. Um auch eine wirksame Behandlung des Teilraumes 4 mit den Behandlungsmedien sicher zu stellen, weist der Düsenkörper 8 an seiner Umfangsfläche weitere, ebenfalls mit dem Düsenkanal 8.1 in Verbindung stehende Düsenöffnungen 10 auf, aus denen eine Teilmenge der dem Düsenkanal 8.1 zugeführten Behandlungsmedien in einer Richtung radial oder etwa radial zur Achse des Düsenkörpers 8 austritt und dadurch in den von dem Handgriff 5 gebildeten Teilraum 4 gelangt, sodass auch dieser zuverlässig sterilisiert wird.

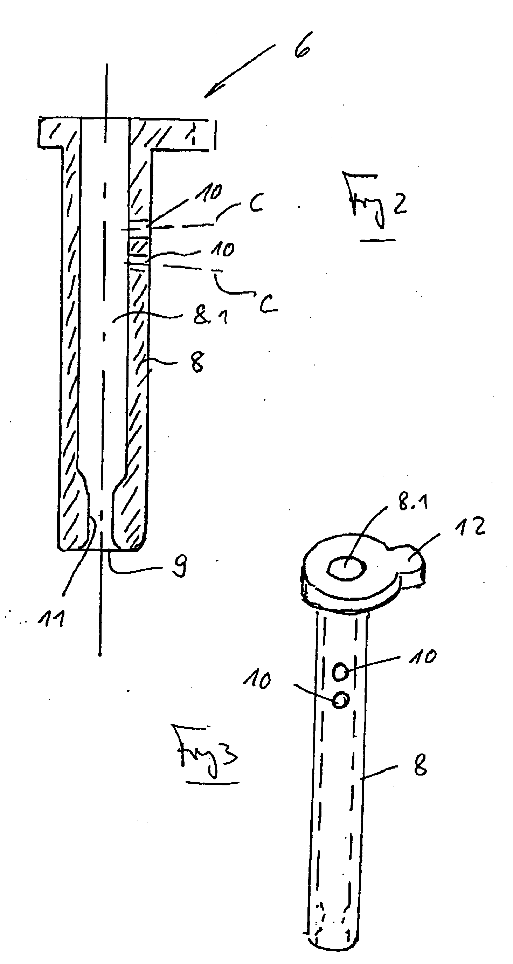

Um sicher zu stellen, dass durch die Düsenöffnungen 10 eine ausreichende Menge an Behandlungsmedium austritt, weist der Düsenkanal 8.1 in Strömungsrichtung auf die Düsenöffnungen 10 folgend eine Verengung auf, wie dies in der Figur 2 bei 11 angedeutet ist.

Weiterhin sind während des Sterilisationsprozesses die Flasche 1 und die Düse 6 relativ zueinander so ausgerichtet, dass sich die Düsenöffnungen 10 bzw. deren Achsen auf denjenigen Bereich der Innenfläche der Flasche 1 gerichtet sind, an dem der Teilraum 4 mit seinem oberen, der Flaschenmündung 2 näher liegenden Ende in den Flaschenraum 3 mündet. Um diese Orientierung zu gewährleisten, werden die Flaschen 1 über ein nicht dargestelltes Transportsystem für den Sterilisationsprozess jeweils in einer vorbestimmten Orientierung bereitgestellt. Weiterhin ist auch die Düse 6 beispielsweise durch eine spezielle Formgebung ihres Düsenkörpers 8 so ausgeführt, dass sie nur in der für die Behandlung der Flaschen 1 notwendigen Orientierung an einem Düsenträger der für den Sterilisationsprozess verwendeten Vorrichtung 7 befestigt werden kann. Entsprechend der Figur 3 weist der Düsenkörper 8 hierfür an seinem oberen Ende eine Ausrichthilfe in Form einer radial weg stehenden Lasche 12 auf.

Die Düsenöffnung 9 ist während des Sterilisationsprozesses mit ihrer Achse auf den Boden der Flasche 1 bzw. des Flascheninnenraumes orientiert, sodass das aus dieser Düsenöffnung austretende Behandlungsmedium auf den Boden auftrifft und sich dann zumindest teilweise in optimaler Weise an den Innenflächen des Flaschenraumes 3 nach oben verteilt, wie dies mit den Pfeilen B in der Figur 1 dargestellt ist. Das aus der zusätzlichen Düsenöffnung 10 austretende Behandlungsmedium gelangt in den Teilraum 4 wie dies in der Figur 1 mit den Pfeilen C angedeutet ist, sodass sich insgesamt eine optimale Strömung ergibt, die eine gleichmäßige Beaufschlagung sämtlicher Bereiche der Flascheninnenfläche mit dem jeweiligen Behandlungsmedium sicher stellt.

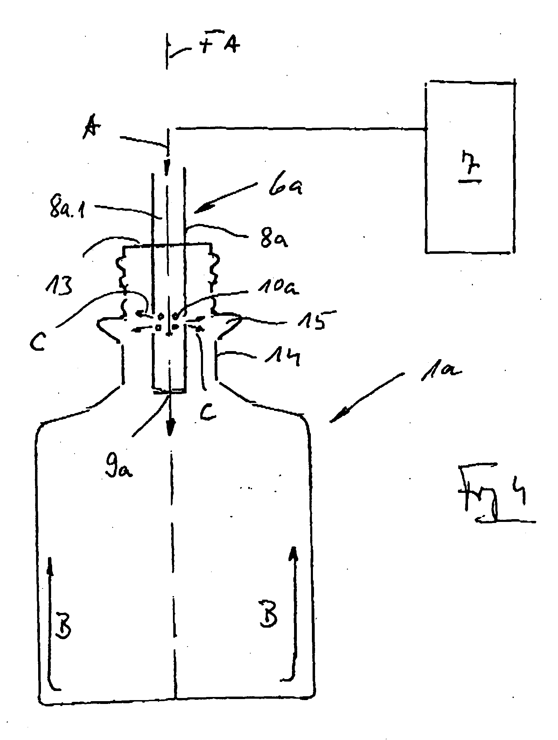

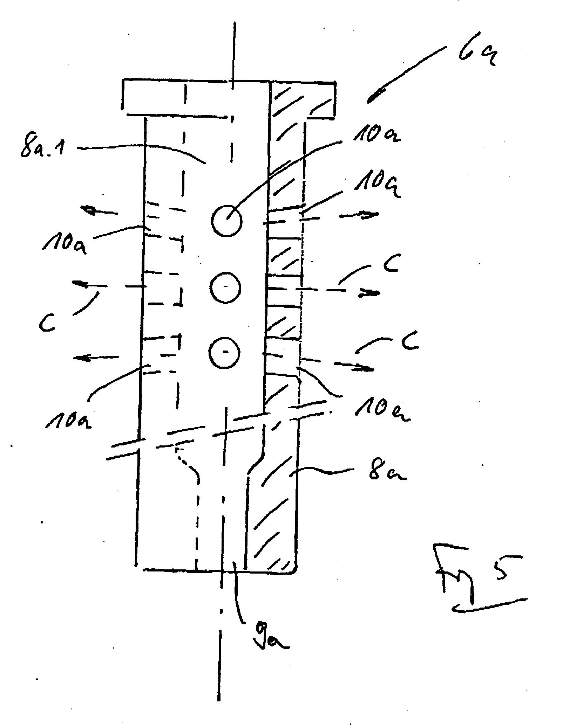

Die Figur 4 zeigt in einer Darstellung ähnlich Figur 1 eine Flasche 1a, die im Bereich ihres an die Flaschenmündung 13 anschließenden Flaschenhalses 14 im Inneren u. a. eine einen Nebenraum 15 bildende Erweiterung aufweist. Um auch diesen die Flaschenachse FA ringförmig umschließenden Neben- oder Teilraum 15 ausreichend mit den Sterilisationsmedien zu behandeln, weist die der Düse 6 entsprechende Düse 6a an der Umfangs- oder Mantelfläche ihres rohrförmigen Düsenkörpers 8a mehrere zusätzliche Düsenöffnungen 10a auf. Diese sind bei der dargestellten Ausführungsform in mehreren, die Achse des Düsenkörpers 8a jeweils ringförmig umschließenden und in der Achsrichtung des Düsenkörpers 8a axial versetzten Gruppen vorgesehen, und zwar derart, dass jede Gruppe mehrere Düsenöffnungen 10a aufweist, die dann beispielsweise in gleichmäßigen Winkelabständen um die Achse des Düsenkörpers 8a verteilt vorgesehen sind. Die Düsenöffnungen 10a der einzelnen Gruppen sind beispielsweise mit ihrer Achse in unterschiedlicher Achsrichtung orientiert, wie dies in der Figur 5 dargestellt ist, sodass nicht nur der Nebenraum 15 durch die diesem Nebenraum unmittelbar gegenüberliegenden, mit ihren Düsenöffnungen 10a, sondern auch andere, mit einer Profilierung oder einer sich ändernden Kontur ausgebildete Bereiche der Innenfläche des Flaschenhalses 14 in optimaler Weise mit den Behandlungsmedien beaufschlagt werden. Die Düsenöffnungen 10a sind beispielsweise mit einem runden oder ovalen Querschnitt oder schlitzförmig ausgebildet.

Um auch bei dieser Ausführung sicher zu stellen, dass eine ausreichende Menge des jeweiligen Behandlungsmediums aus den Düsenöffnungen 10a austritt, ist der im Düsenkörper 8a ausgebildete Düsenkanal 8a.1 wiederum in Strömungsrichtung nach den Düsenöffnungen 10a verengt oder aber die der Düsenöffnung 9 entsprechende Düsenöffnung 9a am unteren Ende des Düsenkörpers 8a besitzt einen gegenüber dem Querschnitt des Düsenkanals 8a.1 reduzierten Querschnitt, wie dies in der Figur 5 dargestellt ist.

Die Erfindung wurde voranstehend an Ausführungsbeispielen beschrieben. Es versteht sich, dass Änderungen sowie Abwandlungen möglich sind, ohne dass dadurch der die Erfindung tragende Gedanke verlassen wird.

Allen Ausführungen der Erfindung ist gemeinsam, dass während des Sterilisationsprozesses über zusätzliche Düsenöffnungen das jeweilige Behandlungsmedium gezielt auch in durch die Behälterform gebildete Erweiterungen und/oder Nebenräume des Behälter oder Flascheninnenraumes eingebracht wird, d. h. auch die dortigen Behälterinnenflächen mit dem jeweiligen Behandlungsmedium beaufschlagt werden, die Behandlung der Behälter bzw.

Flaschen also mit Behandlungsdüsen erfolgt, die hinsichtlich Anordnung und Anzahl der Düsenöffnungen an die Innenkontur der Behälter bzw. Flaschen angepasst sind und die somit eine optimale Beaufschlagung aller Bereiche der Innenfläche eines Behälters oder einer Flasche mit dem jeweiligen Behandlungsmedium bzw. eine optimale Strömung des Behandlungsmediums während des Sterilisationsprozesses sicherstellen.

Vorstehend wurde der einfacheren Darstellung wegen davon ausgegangen, dass die Düsenöffnungen durch Ausströmöffnungen gebildet sind. Grundsätzlich ist es auch möglich, spezielle Düsenelemente einzusetzen, die dann jeweils eine oder eventuell mehrere Düsenaustrittsöffnungen bilden, und zwar z. B. auch mit einer speziellen Düsen- oder Strömungscharakteristik.

### Bezugszeichenliste

- 1, 1a: Flasche
- 2: Flaschenmündung
- 3: Flascheninnenraum
- 4: Teilraum
- 5: Handgriff
- 6, 6a: Behandlungsdüse
- 7: Vorrichtung
- 8, 8a: Düsenkörper
- 8.1, 8a.1: Düsenkanal
- 9,9a: Düsenöffnung
- 10, 10a: Düsenöffnung
- 11: Verengung
- 12: Ausrichthilfe bzw. Lasche
- 13: Flachenmündung
- 14: Flaschenhals
- 15: Nebenraum

- A: Strömungsrichtung des zugeführten Behandlungsmediums
- B, C: Strömungsrichtung des Behandlungsmediums innerhalb der Flasche

## Patentansprüche

1. Verfahren zum Sterilisieren von Flaschen oder dergleichen Behälter (1, 1 a), welche einen Innenraum (3) und einen in Umfangsrichtung nicht symmetrisch angeordneten Teil- oder Nebenraum (4) aufweisen, unter Verwendung wenigstens eines Sterilisations- oder Behandlungsmediums, welches über wenigstens eine in den jeweiligen Behälter (1, 1a) eingeführte Behandlungsdüse (6, 6a) auf die Behälterinnenfläche aufgebracht wird, wobei die Behandlungsdüse (6, 6a) mehrere Düsenöffnungen (9, 9a; 10, 10a) aufweist, die hinsichtlich ihrer Anzahl und/oder Anordnung an die Innenkontur des jeweiligen Behälters angepasst sind, wobei die eine Düsenöffnung (9, 9a) in Achsrichtung des Behälters (1, 1a) und die andere Gruppe der Düsenöffnungen (10, 10a) quer zur Behälterachse (FA) ausgerichtet ist, und Düsenöffnung (9, 9a) beim Einführen der Behandlungsdüse (6, 6a) in den jeweiligen Behälter (1, 1 a) zuerst in den Innenraum (3) eintritt, **dadurch gekennzeichnet, dass**
zur Behandlung von Behältern mit einem in Umfangsrichtung nicht symmetrisch angeordneten Teil- oder Nebenraum (4) bildenden Innenkontur die jeweiligen Behälter und die Behandlungsdüse (6, 6a) relativ zueinander derart ausgerichtet werden, dass anschließend die ebenfalls am Düsenkanals (8) angeordnete wenigstens eine weitere Düsenöffnung (10, 10a) in den Behälter eintritt, wobei die mindestens eine Düsenöffnung (10, 10a) mit ihrer Wirkungsachse nur auf den in Umfangsrichtung nicht symmetrisch angeordneten Teil- oder Nebenraum (4) bzw. dessen Öffnung zum Innenraum gerichtet ist.

2. Verfahren nach einem der vorhergehenden Ansprüche, **dadurch gekennzeichnet, dass** das wenigstens eine Behandlungsmedium aus der wenigstens einen weiteren Düsenöffnung (10, 10a) in einem Winkel zur Behälterachse (FA) ausgebracht wird.

3. Verfahren nach einem der vorhergehenden Ansprüche, **dadurch gekennzeichnet, dass** das wenigstens ein Behandlungsmedium Wasserstoffperoxid enthält, beispielsweise ein Aerosol aus steriler Luft und Wasserstoffperoxid ist.

4. Verfahren nach einem der vorhergehenden Ansprüche, **dadurch gekennzeichnet, dass** das wenigstens eine Behandlungsmedium heiße sterile Luft ist.

5. Vorrichtung zum Sterilisieren von Flaschen oder dergleichen Behälter), welche einen Innenraum (3) und einen in Umfangsrichtung nicht symmetrisch angeordneten Teil- oder Nebenraum (4) aufweisen, mit wenigstens einer Behandlungsdüse (6, 6a), welche während des Sterilisationsprozesses in den jeweiligen Behälter (1, 1 a) zum Aufbringen eines Sterilisations- oder Behandlungsmediums auf die Behälterinnenflächen eingeführt ist, wobei die Behandlungsdüse (6, 6a) mehrere Düsenöffnungen (9, 9a; 10, 10a) auf unterschiedlichen Ebenen entlang der Achse der Behandlungsdüse (6, 6a) aufweist, die hinsichtlich ihrer Anzahl und/oder Anordnung an die Innenkontur des jeweiligen Behälters adaptiert sind, **dadurch gekennzeichnet, dass** zur Behandlung von Behältern (1, 1a) mit einer in Umfangsrichtung nicht symmetrisch angeordneten wenigstens einen Teil- oder Nebenraum (4) bildenden Innenkontur, der Düsenkörper (6, 6a) am freien Ende des Düsenkörpers (8) mindestens eine Düsenöffnung (9) aufweist und in einer vom freien Ende in Achsrichtung beabstandeten Ebene oder Bereich weitere Düsenöffnungen (10) angeordnet sind, die in einem begrenzten Winkelbereich seitlich aus dem Düsenkörper herausführen, so dass im bestimmungsgemäßen Betrieb die Düsenöffnungen (10) nur auf den in Umfangsrichtung nicht symmetrisch angeordneten Teil- oder Nebenraum (4) bzw. dessen Öffnung zum Innenraum (3) hin gerichtet sind.

6. Vorrichtung nach Anspruch 7, **dadurch gekennzeichnet, dass** die Behandlungsdüse (6, 6a) an ihrem Düsenkörper (8, 8a) wenigstens zwei Düsenöffnungen (9, 9a) aufweist, die mit ihren Wirkachsen in unterschiedlichen Richtungen orientiert sind.

7. Vorrichtung nach Anspruch 7 oder 8, **dadurch gekennzeichnet, dass** die Behandlungsdüse (6, 6a) wenigstens eine Düsenöffnung aufweist, die während des Sterilisationsprozesses mit ihrer Wirkachse auf den Boden des jeweiligen Behälters gerichtet ist.

8. Vorrichtung nach einem der vorhergehenden Ansprüche, **dadurch gekennzeichnet, dass** am Umfang des Düsenkörper (8) mehrere Düsenöffnungen (10, 10a) vorgesehen sind, die mit ihrer Wirkachse jeweils unterschiedlich orientiert sind.

## Claims

1. Method for sterilizing bottles or similar containers (1, 1 a), which have an interior space (3) and a part or secondary space (4) that is positioned in a non symmetrical manner in the circumferential direction, by using at least one sterilization or treatment medium, which is applied onto the container inside surface by means of at least one treatment nozzle (6, 6a) that is introduced into the respective container (1, 1a), wherein the treatment nozzle (6, 6a) has a plurality of nozzle openings (9, 9a; 10, 10a), which, with regard to their number and/or arrangement, are adapted to the inside contour of the respective container, wherein the one nozzle opening (9, 9a) is aligned in the axial direction of the container (1, 1a) and the other group of nozzle openings (10, 10a) is aligned transversely relative to the container axis (FA), and nozzle opening (9, 9a) only enters the interior space (3) when the treatment nozzle (6, 6a) is introduced into the respective container (1, 1a), **characterized in that** for treating containers with an inside contour forming a part or secondary space (4) that is positioned in a non symmetrical manner in the circumferential direction, the respective containers and the treatment nozzle (6, 6a) are aligned relative to one another in such a manner that subsequently the at least one additional nozzle opening (10, 10a) that is also positioned at the nozzle channel (8) enters into the container, wherein the at least one nozzle opening (10, 10a) is directed with its effective axis only onto the part or secondary space (4) positioned in a non-symmetrical manner in the circumferential direction or onto its opening to the interior space.

2. Method according to one of the preceding Claims, **characterized in that** at least one treatment medium is dispensed from the at least one additional nozzle opening (10, 10a) at an angle relative to the container axis (FA).

3. Method according to one of the preceding Claims, **characterized in that** at least one treatment medium contains hydrogen peroxide, for example an aerosol made from sterile air and hydrogen peroxide.

4. Method according to one of the preceding Claims, **characterized in that** the at least one treatment medium is hot sterile air.

5. Device for sterilizing bottles or similar containers, which have an interior space (3) and a part or secondary space (4) that is positioned in a non-symmetrical manner in the circumferential direction, said device having at least one treatment nozzle (6, 6a), which is introduced into the respective container (1, 1a) during the sterilization process for dispensing a sterilization or treatment medium onto the container inside surfaces, wherein the treatment nozzle (6, 6a) has a plurality of nozzle openings (9, 9a; 10, 10a) on different planes along the axis of the treatment nozzle (6, 6a), said nozzle openings, with regard to their number and/or arrangement, being adapted to the inside contour of the respective container, **characterized in that** for treating containers (1, 1 a) with an inside contour forming at least one part or secondary space (4) that is positioned in a non-symmetrical manner in the circumferential direction, the nozzle body (6, 6a) has at least one nozzle opening (9) at the free end of the nozzle body (8) and additional nozzle openings (10) are positioned in a plane or region at a spacing in the axial direction from the free end, said nozzle openings extending laterally from the nozzle body in a restricted angular region such that, in normal operation, the nozzle openings (10) are only directed onto the part or secondary space (4) positioned in a non-symmetrical manner in the circumferential direction or onto its opening to the interior space (3).

6. Device according to Claim 7, **characterized in that** the treatment nozzle (6, 6a) has at least two nozzle openings (9, 9a) on its nozzle body (8, 8a), said nozzle openings being orientated with their effective axes in different directions.

7. Device according to Claim 7 or 8, **characterized in that** the treatment nozzle (6, 6a) has at least one nozzle opening that is directed with its effective axis onto the bottom of the respective container during the sterilization process.

8. Device according to one of the preceding Claims, **characterized in that** a plurality of nozzle openings (10, 10a) are provided on the periphery of the nozzle body (8), each of said nozzle openings being oriented with their effective axis in a different manner.

## Revendications

1. Procédé de stérilisation de bouteilles ou récipients (1, 1 a) similaires qui comportent un volume intérieur (3) et une partie ou volume secondaire (4), disposé non symétriquement dans la direction périphérique, moyennant l'utilisation d'au moins un fluide de stérilisation ou fluide de traitement, qui est déposé sur la face intérieure du récipient par l'intermédiaire d'au moins une buse de traitement (6, 6a) introduite dans le récipient (1, 1a) concerné, ladite buse de traitement (6, 6a) comportant plusieurs ouvertures (9, 9a ; 10, 10a), qui par leur nombre et/ou leur agencement sont adaptées au contour intérieur du récipient concerné, l'une des ouvertures de buse (9, 9a) étant orientée dans la direction axiale du récipient (1, 1a) et l'autre groupe d'ouvertures de buse (10, 10a) étant orienté transversalement à l'axe (FA) du récipient, l'ouverture de buse (9, 9a) pénétrant en premier dans le volume intérieur (3) au moment de l'introduction de la buse de traitement (6, 6a) dans le récipient (1, 1a) concerné, **caractérisé en ce que**
pour le traitement des récipients avec un contour intérieur formant la partie ou volume secondaire (4) disposé non symétriquement dans la direction périphérique, les récipients concernés et la buse de traitement (6, 6a) sont orientés les uns par rapport aux autres de telle sorte que ladite au moins une ouverture de buse (10, 10a) supplémentaire, également réalisée sur le canal (8) de la buse, pénètre ensuite dans le récipient, ladite au moins une ouverture de buse (10, 10a) étant dirigée avec son axe de travail seulement sur la partie ou volume secondaire (4), disposé non symétriquement dans la direction périphérique, plus précisément sur l'ouverture de celui-ci vers le volume intérieur.

2. Procédé selon l'une des revendications précédentes, **caractérisé en ce que** ledit au moins un fluide de traitement est distribué par l'intermédiaire de ladite au moins une ouverture de buse (10, 10a) supplémentaire en formant un angle avec l'axe (FA) du récipient.

3. Procédé selon l'une quelconque des revendications précédentes, **caractérisé en ce que** ledit au moins un fluide de traitement contient du peroxyde d'hydrogène, tel qu'un aérosol formé d'air stérile et de peroxyde d'hydrogène.

4. Procédé selon l'une quelconque des revendications précédentes, **caractérisé en ce que** ledit au moins un fluide de traitement est de l'air stérile chaud.

5. Dispositif de stérilisation de bouteilles ou récipients (1, 1 a) similaires qui comportent un volume intérieur (3) et une partie ou volume secondaire (4), disposé non symétriquement dans la direction périphérique, comportant au moins une buse de traitement (6, 6a) qui, pendant le processus de stérilisation, est introduite dans le récipient (1, 1a) concerné en vue de déposer un fluide de stérilisation ou fluide de traitement sur les faces intérieures des récipients, ladite buse de traitement (6, 6a) comportant plusieurs ouvertures (9, 9a ; 10, 10a) sur différents plans le long de l'axe de la buse de traitement (6, 6a), lesquelles par leur nombre et/ou leur agencement sont adaptées au contour intérieur du récipient concerné, **caractérisé en ce que** pour le traitement des récipients (1, 1a) avec un contour intérieur formant la partie ou volume secondaire (4) disposé non symétriquement dans la direction périphérique, le corps de buse (6, 6a) au niveau de l'extrémité libre du corps de buse (8) comporte au moins une ouverture de buse (9), et dans un plan ou zone éloigné de l'extrémité libre dans la direction axiale sont disposées des ouvertures de buse (10) supplémentaires, qui sortent latéralement du corps de buse selon une zone angulaire limitée, de telle sorte que, en cours de service conforme à la destination, des ouvertures de buse (10) sont dirigées seulement sur la partie ou volume secondaire (4), disposé non symétriquement dans la direction périphérique, plus précisément sur l'ouverture de celui-ci vers le volume intérieur.

6. Dispositif selon la revendication 1, **caractérisé en ce que** la buse de traitement (6, 6a), sur son corps de buse (8, 8a), comporte au moins deux ouvertures de buse (9, 9a), qui sont orientées avec leur axe de travail dans des directions différentes.

7. Dispositif selon la revendication 5 ou 6, **caractérisé en ce que** la buse de traitement (6, 6a) comporte au moins une ouverture de buse qui, pendant le processus de stérilisation, est dirigée avec son axe de travail sur le fond du récipient concerné.

8. Dispositif selon l'une quelconque des revendications précédentes, **caractérisé en ce que** sur le pourtour du corps de buse (8) sont prévues plusieurs ouvertures de buse (10, 10a) qui sont orientées chacune différemment avec leur axe de travail.
